# FASCICULE DE BREVET EUROPEEN

(11) **EP 0 680 749 B1**
(45) Date de publication et mention de la délivrance du brevet: **09.09.1998**
(21) Numéro de dépôt: 95400750.6
(22) Date de dépôt: 04.04.1995
(51) Int. Cl.: A61K 7/48, A61K 38/00

(54) **Utilisation d'un antagoniste de substance P dans une composition cosmétique et composition obtenue**
Verwendung eines Antagonisten der Substanz P in einer kosmetischen Zusammensetzung und erhaltene Zusammensetzung
Cosmetic composition comprising an antagonist of substance P

(30) Priorité: 05.05.1994 FR 9405537
(43) Date de publication de la demande: 08.11.1995
(73) Titulaire: L'OREAL, 75008 Paris (FR)
(72) Inventeur: de Lacharriere, Olivier, F-75015 Paris (FR); Breton, Lionel, F-78000 Versailles (FR)
(74) Mandataire: Lhoste, Catherine

(56) Documents cités:
- EP-A- 0 299 457
- EP-A- 0 360 390
- EP-A- 0 429 366
- EP-A- 0 514 273
- EP-A- 0 517 589
- EP-A- 0 520 555
- EP-A- 0 522 808
- EP-A- 0 528 495
- EP-A- 0 545 478
- WO-A-83/01252
- WO-A-90/05729
- WO-A-91/18899
- WO-A-93/01159
- WO-A-93/01170
- WO-A-93/14084
- GB-A- 2 271 774
- BR. J. PHARMACOL., vol. 109, no. 1, 1993, pages 259-264, XP002008594 S.M. MOUSSAOUI: "A non-peptide NK1-receptor antagonist, RP 67580, inhibits neurogenic inflammation postsynaptically"
- BR. J. DERMATOL., vol. 124, no. 4, 1991, pages 324-328, XP002008595 J. WALLENGREN: "Substance P antagonist inhibits immediate and delayed type cutaneous hypersensitivity reactions"
- CONTACT DERMATITIS, vol. 19, no. 5, 1988, pages 351-354, XP002008596 J. WALLENGREN: "Some neuropeptides as modulators of experimental contact allergy"
- J. AM. ACAD. DERMATOL., vol. 30, no. 2PT1, Février 1994, pages 232-235, XP002008597 T. LOTTI: "Treatment of aquagenic pruritus with topical capsaicin cream"
- BRAIN RES., vol. 593, no. 2, 1992, pages 319-322, XP002008598 T. SAKURADA: "A selective and extremely potent antagonist of the neurokinin-1 receptor"
- PROC. NATL. ACAD. SCI. USA, vol. 87, no. 12, 1990, pages 4833-4855, XP002008599 K. FOLKERS: "Spantide II, an efective tachychinin antagonist having high potency and negligible neurotoxicity"

## Description

La présente invention concerne l'utilisation d'un antagoniste de substance P dans une composition cosmétique, pour traiter les peaux sensibles, ainsi que la composition cosmétique obtenue.

Il est connu que certaines peaux sont plus sensibles que d'autres. Les symptômes des peaux sensibles étaient jusqu'à présent mal caractérisés et le problème de ces peaux était, de ce fait, mal défini personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques l'emploi de produits à caractère irritant tels que les tensioactifs, les conservateurs ou les parfums.

La demanderesse a réalisé de nombreux tests cliniques et elle a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a maintenant trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments. En général, ces signes sont associés à une peau hyperséborrhéique ou acnéique avec ou sans dartres, et à un érythème.

Les cuirs chevelus "sensibles" ont une sémiologie clinique plus univoque les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Cette substance P est un élément chimique polypeptidique élaboré et libéré par une terminaison nerveuse. Elle fait partie de la famille des tachykinines. La substance P intervient notamment dans la transmission de la douleur et dans des maladies du système nerveux central tels que l'anxiété, la schizophrénie, dans des maladies respiratoires et inflammatoires, dans des maladies gastrointestinales, dans des maladies rhumatismales et dans certaines maladies dermatologiques telles que l'eczéma.

La demanderesse a maintenant découvert que la caractéristique essentielle des peaux sensibles était liée à la libération de substance P et donc que l'utilisation d'antagonistes de substance P pouvait permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition cosmétique permettait d'éviter l'irritation et/ou les sensations dysesthésiques et/ou les prurits de la peau.

La présente invention a donc pour objet l'utilisation d'un antagoniste de substance P dans une composition contenant un milieu cosmétiquement acceptable, pour traiter les peaux sensibles.

La présente invention a encore pour objet l'utilisation d'un antagoniste de substance P pour prévenir et/ou lutter contre les irritations cutanées et/ou les dartres et/ou les érythèmes et/ou les sensations d'échauffement et/ou de dysesthésie et/ou les prurits de la peau.

Un milieu cosmétiquement acceptable est un milieu qui est compatible avec la peau, les ongles et les cheveux. La composition contenant l'antagoniste de subtance P peut être appliquée sur le visage, le cou, les cheveux et les ongles, ou toute autre zone cutanée du corps.

Pour qu'une substance soit reconnue comme un antagoniste de substance P, elle doit répondre aux caractéristiques suivantes :
- avoir une affinité sélective pour les récepteurs NK1 des tachykinines,
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induite par l'administration de substance P.

Jusqu'à ce jour, les antagonistes de substance P étaient utilisés pour traiter les maladies indiquées ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808 et WO-A-93/01165.

Personne jusqu'à ce jour n'avait établi un lien entre la substance P et la peau sensible. Les signes cliniques de la peau sensible sont essentiellement subjectifs: picotements, fourmillements, prurits, tiraillements, échauffements, et ils s'associent parfois à des érythèmes. Ces signes sont dûs à des facteurs extérieurs aspécifiques. Les symptômes apparaissent essentiellement localisés au visage, au cou et au cuir chevelu, mais peuvent apparaître aussi sur tout le corps.

L'antagoniste de substance P de l'invention peut être un peptide ou un dérivé azoté non peptidique, et plus précisément un composé comportant un hétérocycle azoté ou un atome d'azote lié directement ou indirectement à un cycle benzènique.

On peut utiliser dans l'invention par exemple comme peptide antagoniste de substance P le sendide et le spantide II.

Le sendide correspond à la formule :
Tyr D-Phe Phe D-His Leu Met NH₂
dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :
D-NicLys Pro 3-Pal Pro D-Cl₂Phe Asn D-Trp Phe D-Trp Leu Nle NH₂
dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un atome d'azote lié directement ou indirectement à un cycle benzénique ou contenu dans un hétérocycle.

Comme composé hétérocyclique, on peut utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116. En particulier, le composé comprenant au moins un hétérocycle azoté est un dérivé de 2-tricyclyl-2-aminoéthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme composés comportant un atome d'azote lié directement ou indirectement à un noyau benzénique, on peut citer ceux décrits dans les documents suivants: EP-A-522808 et WO-A-93/01165.

Dans les compositions selon l'invention, l'antagoniste de substance P est utilisé de préférence en une quantité allant de 0,000001 à 5 % en poids par rapport au poids total de la composition, et en particulier en une quantité allant de 0,0001 à 0,1 % en poids par rapport au poids total de la composition.

Les compositions selon l'invention peuvent se présenter sous toutes les formes galéniques normalement utilisées pour une application topique, notamment des solutions ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou émulsions de consistance molle du type crème ou gel, ou encore des microgranulés, ou des dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

L'antagoniste de substance P peut être aussi incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions cosmétiques de l'invention peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 30 % en poids par rapport au poids total de la composition. L'émulsion peut, en outre, contenir des vésicules lipidiques.

De façon connue, la composition cosmétique de l'invention peut contenir également des adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser les protéines ou les hydrolysats de protéine, les acides aminés, les polyols, I'urée, I'allantoïne, les sucres et les dérivés de sucre, les vitamines et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

On peut entre autres associer les antagonistes de substance P à des agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, I'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, I'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

De façon avantageuse, les antagonistes de substance P sont associés à des actifs à effet secondaire irritant utilisés couramment dans le domaine cosmétique. La présence d'un antagoniste dans une composition cosmétique contenant un actif ayant un effet irritant permet d'atténuer fortement, voire de supprimer cet effet irritant.

Aussi, l'invention a encore pour objet une composition contenant un milieu cosmétiquement acceptable et au moins un actif à effet secondaire irritant, caractérisée en ce qu'elle contient un antagoniste de substance P.

En particulier, les actifs à effet secondaire irritant sont choisis parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés.

La présente invention a en outre pour objet un procédé de traitement cosmétique, caractérisé par le fait que l'on applique sur la peau, sur les cheveux, et/ou sur les muqueuses, une composition telle que décrite ci-dessus contenant au moins un antagoniste de substance P dans un milieu cosmétiquement acceptable.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

L'invention a encore pour objet l'utilisation de la capsaïcine pour préparer une composition destinée à déterminer les peaux sensibles, ainsi qu'un procédé pour déterminer les peaux sensibles, consistant à appliquer sur la peau une composition contenant de la capsaïcine.

Les exemples suivants illustrent l'invention. Dans ces exemples, les proportions indiquées sont des pourcentages en poids.

### Exemple 1 : lotion démaquillante pour le visage

| | |
|---|---|
| Spantide II | 5,00 |
| Antioxydant | 0,05 |
| Isopropanol | 40,00 |
| Conservateur | 0,30 |
| Eau | qsp 100 % |

### Exemple 2: lotion démaquillante pour le visage

| | |
|---|---|
| Sendide | 0,0001 |
| Antioxydant | 0,05 |
| Isopropanol | 40,00 |
| Conservateur | 0,30 |
| Eau | qsp 100 % |

### Exemple 3 : Gel pour le soin du visage

| | |
|---|---|
| Spantide II | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

### Exemple 4 : Gel pour le soin du visage

| | |
|---|---|
| Sendide | 0,04 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

### Exemple 5 : Crème de soin du visage (émulsion huile dans eau)

| | |
|---|---|
| Spantide II | 0,02 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 6 : Shampooing

| | |
|---|---|
| Spantide II | 0,02 |
| Hydroxypropylcellulose (Klucel H vendu par la société Hercules) | 1,00 |
| Parfum | 0,50 |
| Conservateur | 0,30 |
| Eau | qsp 100 % |

### Exemple 7 : Crème de soin antirides pour le visage (émulsion huile dans eau)

| | |
|---|---|
| Sendide | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 8 : Shampooing

| | |
|---|---|
| Sendide | 0,003 |
| Lauryl éther sulfate de sodium et magnésium à 4 moles d'oxyde d'éthylène, vendu sous le nom de TEXAPON ASV par HENKEL (tensio-actif anionique) | 6,5 g |
| Hydroxypropylcellulose (Klucel H vendu par la société Hercules) | 1,00 |
| Parfum | 0,50 |
| Conservateur | 0,30 |
| Eau | qsp 100 % |

### Exemple 9 : Gel émulsionné de soin contre les piqûres d'insectes (émulsion huile dans eau)

| | |
|---|---|
| Cyclomethicone | 3,00 |
| Huile de Purcellin (vendue par la Société Dragocco) | 7,00 |
| PEG-6/PEG-32/Glycol Stéarate (Tefose^{R} 63 de Gattefosse) | 0,30 |
| Spantide II | 0,02 |
| Conservateur | 0,30 |
| Parfum | 0,40 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100 % |

### Exemple 10 : Gel anti-douleur

| | |
|---|---|
| Spantide II | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

### Exemple 11 : Crème de soin de la rosacée pour le visage (émulsion huile dans eau)

| | |
|---|---|
| Spantide II | 0,25 |
| Stéarate de glycérol | 2,00 |
| Polysorbate 60 (Tween 60 vendu par la société ICI) | 1,00 |
| Acide stéarique | 1,40 |
| Métronidazole | 1,00 |
| Triéthanolamine | 0,70 |
| Carbomer | 0,40 |
| Fraction liquide du beurre de karité | 12,00 |
| Huile de vaseline | 12,00 |
| Antioxydant | 0,05 |
| Parfum | 0,5 |
| Conservateur | 0,30 |
| Eau | qsp 100 % |

### Exemple 12 : Crème de soin des peaux sensibles contre l'érythème solaire (émulsion huile dans eau)

| | |
|---|---|
| Spantide II | 0,25 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

## Revendications

1. Utilisation d'un antagoniste de substance P dans une composition contenant un milieu cosmétiquement acceptable pour traiter les peaux sensibles.

2. Utilisation selon la revendication 1, caractérisée en ce que l'antagoniste de substance P est une substance qui diminue l'extravasation du plasma au travers de la paroi vasculaire induite par la capsaïcine ou par une stimulation nerveuse antidromique.

3. Utilisation selon la revendication 1 ou 2, caractérisée en ce que l'antagoniste de substance P est une substance qui provoque une inhibition de la contraction des muscles lisses induites par l'administration de substance P.

4. Utilisation selon l'une quelconque des revendications précédentes, caractérisée en ce que l'antagoniste de substance P est choisi parmi les peptides et les composés comprenant au moins un hétérocycle azoté.

5. Utilisation selon la revendication précédente, caractérisée en ce que le peptide est le sendide ou le spantide II.

6. Utilisation selon la revendication 4, caractérisée en ce que le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

7. Utilisation selon l'une quelconque des revendications précédentes, caractérisée en ce que l'antagoniste de substance P est utilisé en une quantité allant de 0,000001 à 5 % en poids par rapport au poids total de la composition.

8. Utilisation selon l'une quelconque des revendications précédentes, caractérisée en ce que l'antagoniste de substance P est utilisé en une quantité allant de 0,0001 à 0,1 % en poids par rapport au poids total de la composition.

9. Utilisation selon l'une quelconque des revendications précédentes, caractérisée en ce que le milieu cosmétiquement acceptable est une solution aqueuse ou hydroalcoolique, une émulsion eau-dans-huile, une émulsion huile-dans-eau, une microémulsion, un gel aqueux, un gel anhydre, un sérum, une dispersion de vésicules.

10. Utilisation selon l'une quelconque des revendications précédentes, caractérisée en ce que la composition contient au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

11. Utilisation selon la revendication précédente, caractérisée en ce que l'agent est le chlorhydrate de lidocaïne, un antiparasitaire ou un anti-inflammatoire non stéroïdien.

12. Utilisation dans une composition cosmétique d'un antagoniste de substance P pour prévenir et/ou lutter contre les irritations cutanées et/ou les dartres et/ou les érythèmes et/ou les sensations dysesthésiques et/ou les sensations d'échauffement et/ou les prurits de la peau.

13. Utilisation selon la revendication 12, caractérisée en ce que l'antagoniste de substance P est une substance qui diminue l'extravasation du plasma au travers de la paroi vasculaire induite par la capsaïcine ou par une stimulation nerveuse antidromique.

14. Utilisation selon la revendication 12 ou 13, caractérisée en ce que l'antagoniste de substance P est une substance qui provoque une inhibition de la contraction des muscles lisses induites par l'administration de substance P.

15. Utilisation selon l'une quelconque des revendications 12 à 14, caractérisée en ce que l'antagoniste de subtance P est choisi parmi les peptides et les composés comprenant au moins un hétérocycle azoté.

16. Utilisation selon l'une quelconque des revendications 12 à 15, caractérisée en ce que l'antagoniste de substance P est utilisé en une quantité allant de 0,000001 à 5 % en poids par rapport au poids total de la composition.

17. Utilisation selon l'une quelconque des revendications 12 à 16, caractérisée en ce que l'antagoniste de substance P est utilisé en une quantité allant de 0,0001 à 0,1 % en poids par rapport au poids total de la composition.

18. Procédé de traitement cosmétique, caractérisé en ce que l'on applique sur la peau, sur les cheveux et/ou sur les muqueuses des sujets à peaux sensibles une composition contenant un antagoniste de substance P dans un milieu cosmétiquement acceptable.

19. Procédé selon la revendication 18, caractérisé en ce que l'antagoniste de substance P est une substance qui diminue l'extravasation du plasma au travers de la paroi vasculaire induite par la capsaïcine ou par une stimulation nerveuse antidromique.

20. Procédé selon la revendication 18 ou 19, caractérisé en ce que l'antagoniste de substance P est une substance qui provoque une inhibition de la contraction des muscles lisses induites par l'administration de substance P.

21. Procédé selon l'une quelconque des revendications 18 à 20, caractérisé en ce que l'antagoniste de substance P est choisi parmi les peptides et les composés comprenant au moins un hétérocycle azoté.

22. Procédé selon l'une quelconque des revendications 18 à 21, caractérisé en ce que l'antagoniste de substance P est utilisé en une quantité allant de 0,000001 à 5 % en poids par rapport au poids total de la composition.

23. Procédé selon l'une quelconque des revendications 18 à 22, caractérisé en ce que l'antagoniste de substance P est utilisé en une quantité allant de 0,0001 à 0,1 % en poids par rapport au poids total de la composition.

24. Composition cosmétique contenant un milieu cosmétiquement acceptable et au moins un actif à effet secondaire irritant, caractérisée en ce qu'elle contient un antagoniste de substance P.

25. Composition cosmétique selon la revendication 24, caractérisée en ce que l'antagoniste de substance P est une substance qui diminue l'extravasation du plasma au travers de la paroi vasculaire induite par la capsaïcine ou par une stimulation nerveuse antidromique.

26. Composition cosmétique selon la revendication 24 ou 25, caractérisée en ce que l'antagoniste de substance P est une substance qui provoque une inhibition de la contraction des muscles lisses induites par l'administration de substance P.

27. Composition cosmétique selon l'une quelconque des revendications 24 à 26, caractérisée en ce que l'antagoniste de substance P est choisi parmi les peptides et les composés comprenant au moins un hétérocycle azoté.

28. Composition cosmétique selon l'une quelconque des revendications 24 à 27, caractérisée en ce que l'antagoniste de substance P est utilisé en une quantité allant de 0,000001 à 5 % en poids par rapport au poids total de la composition.

29. Composition cosmétique selon l'une quelconque des revendications 24 à 28, caractérisée en ce que l'antagoniste de substance P est utilisé en une quantité allant de 0,0001 à 0,1 % en poids par rapport au poids total de la composition.

30. Composition cosmétique selon l'une quelconque des revendications 24 à 29, caractérisée en ce que l'actif à effet secondaire irritant est choisi parmi les α-hydroxy-acides, les β-hydroxy-acides, les α-céto-acides, les β-céto-acides les rétinoïdes, les anthralines, les anthranoïdes, les peroxydes, le minoxidil, les sels de lithium, les antimétabolites, la vitamine D et ses dérivés, les tensioactifs, les parfums et les conservateurs.

31. Composition cosmétique selon l'une quelconque des revendications 24 à 30, caractérisée en ce qu'elle contient en outre au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

32. Utilisation de la capsaïcine pour préparer une composition destinée à déterminer les peaux sensibles.

33. Composition de nettoyage et/ou de protection et/ou de traitement et/ou de soin pour le visage et/ou les mains et/ou les pieds et/ou les grands plis anatomiques et/ou le corps, et/ou de maquillage et/ou démaquillante et/ou après-solaire et/ou de bronzage et/ou pour le bain et/ou désodorisante et/ou épilatoire et/ou après-rasage et/ou contre les piqûres d'insectes et/ou anti-douleur et/ou de parfum et/ou de shampooing et/ou de soin capillaire et/ou de traitement capillaire et/ou bucco-dentaire, caractérisée en ce qu'elle est conforme à l'une quelconque des revendications 24 à 31.

34. Composition cosmétique selon l'une des revendications 24 à 31 ou 33, caractérisée en ce que le milieu cosmétiquement acceptable est une solution aqueuse ou hydroalcoolique, une émulsion eau-dans-huile, une émulsion huile-dans-eau, une microémulsion, un gel aqueux, un gel anhydre, un sérum, une dispersion de vésicules.

35. Composition cosmétique selon l'une des revendications 24 à 31, 33 ou 34, caractérisée en ce qu'elle contient des adjuvants tels que les gélifiants, hydrophiles ou lipophiles, les actifs hydrophiles ou lipophiles, les antioxydants, les solvants, les charges, les filtres et les matières colorantes.

## Claims

1. Use of a substance P antagonist in a composition containing a cosmetically acceptable medium, for treating sensitive skin.

2. Use according to Claim 1, characterized in that the substance P antagonist is a substance which lowers the extravasation of plasma across the vascular wall, which is induced by capsaicin or by antidromic nerve stimulation.

3. Use according to Claim 1 or 2, characterized in that the substance P antagonist is a substance which brings about inhibition of the contraction of smooth muscle induced by the administration of substance P.

4. Use according to any one of the preceding claims, characterized in that the substance P antagonist is chosen from peptides and compounds comprising at least one nitrogenous heterocycle.

5. Use according to the preceding claim, characterized in that the peptide is sendide or spantide II.

6. Use according to Claim 4, characterized in that the compound comprising at least one nitrogenous heterocycle is a 2-tricyclyl-2-aminoethane derivative, a spirolactam derivative, a quinuclidine derivative, an azacyclic derivative, an aminopyrrolidine derivative, a piperidine derivative, an aminoazaheterocycle or an isoindole derivative.

7. Use according to any one of the preceding claims, characterized in that the substance P antagonist is used in an amount ranging from 0.000001 to 5% by weight relative to the total weight of the composition.

8. Use according to any one of the preceding claims, characterized in that the substance P antagonist is used in an amount ranging from 0.0001 to 0.1% by weight relative to the total weight of the composition.

9. Use according to any one of the preceding claims, characterized in that the cosmetically acceptable medium is an agueous or aqueous-alcoholic solution, a water-in-oil emulsion, an oil-in-water emulsion, a microemulsion, an aqueous gel, an anhydrous gel, a serum or a vesicle dispersion.

10. Use according to any one of the preceding claims, characterized in that the composition contains at least one agent chosen from antibacterial agents, antiparasitic agents, antifungal agents, antiinflammatory agents, antipruriginous agents, anaesthetics, antiviral agents, keratolytic agents, anti-free-radical agents, antiseborrhoeic agents, antidandruff agents, antiacne agents and/or agents which modify skin differentiation and/or proliferation and/or pigmentation.

11. Use according to the preceding claim, characterized in that the agent is lidocaine hydrochloride, an antiparasitic agent or a non-steroidal antiinflammatory agent.

12. Use, in a cosmetic composition, of a substance P antagonist for preventing and/or combating skin irritations and/or dry patches and/or erythema and/or dysaesthetic sensations and/or sensations of heating and/or pruritus of the skin.

13. Use according to Claim 12, characterized in that the substance P antagonist is a substance which lowers the extravasation of plasma across the vascular wall, induced by capsaicin or by antidromic nerve stimulation.

14. Use according to Claim 12 or 13, characterized in that the substance P antagonist is a substance which brings about inhibition of the contraction of smooth muscle induced by the administration of substance P.

15. Use according to any one of Claims 12 to 14, characterized in that the substance P antagonist is chosen from peptides and compounds comprising at least one nitrogenous heterocycle.

16. Use according to any one of Claims 12 to 15, characterized in that the substance P antagonist is used in an amount ranging from 0.000001 to 5% by weight relative to the total weight of the composition.

17. Use according to any one of Claims 12 to 16, characterized in that the substance P antagonist is used in an amount ranging from 0.0001 to 0.1% by weight relative to the total weight of the composition.

18. Cosmetic treatment process, characterized in that a composition containing a substance P antagonist in a cosmetically acceptable medium is applied to the skin, to the hair and/or to mucous membranes of individuals with sensitive skin.

19. Process according to Claim 18, characterized in that the substance P antagonist is a substance which lowers the extravasation of plasma across the vascular wall, induced by capsaicin or by antidromic nerve stimulation.

20. Process according to Claim 18 or 19, characterized in that the substance P antagonist is a substance which brings about inhibition of the contraction of smooth muscle induced by the administration of substance P.

21. Process according to any one of Claims 18 to 20, characterized in that the substance P antagonist is chosen from peptides and compounds comprising at least one nitrogenous heterocycle.

22. Process according to any one of Claims 18 to 21, characterized in that the substance P antagonist is used in an amount ranging from 0.000001 to 5% by weight relative to the total weight of the composition.

23. Process according to any one of Claims 18 to 22, characterized in that the substance P antagonist is used in an amount ranging from 0.0001 to 0.1% by weight relative to the total weight of the composition.

24. Cosmetic composition containing a cosmetically acceptable medium and at least one active agent with an irritant side effect, characterized in that it contains a substance P antagonist.

25. Cosmetic composition according to Claim 24, characterized in that the substance P antagonist is a substance which lowers the extravasation of plasma across the vascular wall, induced by capsaicin or by antidromic nerve stimulation.

26. Cosmetic composition according to Claim 24 or 25, characterized in that the substance P antagonist is a substance which brings about inhibition of the contraction of smooth muscle induced by the administration of substance P.

27. Cosmetic composition according to any one of Claims 24 to 26, characterized in that the substance P antagonist is chosen from peptides and compounds comprising at least one nitrogenous heterocycle.

28. Cosmetic composition according to any one of Claims 24 to 27, characterized in that the substance P antagonist is used in an amount ranging from 0.000001 to 5% by weight relative to the total weight of the composition.

29. Cosmetic composition according to any one of Claims 24 to 28, characterized in that the substance P antagonist is used in an amount ranging from 0.0001 to 0.1% by weight relative to the total weight of the composition.

30. Cosmetic composition according to any one of Claims 24 to 29, characterized in that the active agent with an irritant side effect is chosen from α-hydroxy acids, β-hydroxy acids, α-keto acids, β-keto acids, retinoids, anthralins, anthranoids, peroxides, minoxidil, lithium salts, antimetabolites, vitamin D and derivatives thereof, surfactants, fragrances and preserving agents.

31. Cosmetic composition according to any one of Claims 24 to 30, characterized in that it also contains at least one agent chosen from antibacterial agents, antiparasitic agents, antifungal agents, antiinflammatory agents, antipruriginous agents, anaesthetics, antiviral agents, keratolytic agents, anti-free-radical agents, antiseborrhoeic agents, antidandruff agents, antiacne agents and/or agents which modify skin differentiation and/or proliferation and/or pigmentation.

32. Use of capsaicin to prepare a composition intended for determining sensitive skin.

33. Cleansing and/or protective and/or treatment and/or care composition for the face and/or the hands and/or the feet and/or the major anatomical folds and/or the body, and/or a make-up and/or make-up-removing and/or antisun and/or tanning and/or bath and/or deodorizing and/or hair-removing and/or aftershave and/or insect-repellent and/or pain-relief and/or fragrance and/or shampoo and/or haircare and/or hair-treatment and/or buccodental composition, characterized in that it is in accordance with any one of Claims 24 to 31.

34. Cosmetic composition according to one of Claims 24 to 31 or 33, characterized in that the cosmetically acceptable medium is an aqueous or aqueous-alcoholic solution, a water-in-oil emulsion, an oil-in-water emulsion, a microemulsion, an aqueous gel, an anhydrous gel, a serum or a vesicle dispersion.

35. Cosmetic composition according to one of Claims 24 to 31, 33 or 34, characterized in that it contains adjuvants such as hydrophilic or lipophilic gelling agents, hydrophilic or lipophilic active agents, antioxidants, solvents, fillers, screening agents and dyestuffs.

## Patentansprüche

1. Verwendung eines Antagonisten der Substanz P in einer Zusammensetzung, die ein kosmetisch akzeptables Medium enthält, zur Behandlung von empfindlicher Haut.

2. Verwendung nach Anspruch 1, dadurch gekennzeichnet, daß der Antagonist der Substanz P eine Substanz ist, die die Extravasation von Plasma durch die Gefäßwand vermindert, welche durch Capsaicin oder eine antidrome Nervenstimulierung hervorgerufen wird.

3. Verwendung nach Anspruch 1 oder 2, dadurch gekennzeichnet, daß der Antagonist der Substanz P eine Substanz ist, die eine Hemmung der durch die Verabreichung der Substanz P hervorgerufenen Kontraktion der glatten Muskulatur hervorruft.

4. Verwendung nach einem der vorhergehenden Ansprüche, dadurch gekennzeichnet, daß der Antagonist der Substanz P unter den Peptiden und den Verbindungen, die mindestens einen Stickstoffheterocyclus aufweisen, ausgewählt ist.

5. Verwendung nach dem vorhergehenden Anspruch, dadurch gekennzeichnet, daß das Peptid das Sendide oder Spantide II ist.

6. Verwendung nach Anspruch 4, dadurch gekennzeichnet, daß die Verbindung, die mindestens einen Stickstoffheterocyclus aufweist, ein 2-Tricyclyl-2-aminoethanderivat, Spirolactamderivat, Chinuclidinderivat, azacyclisches Derivat, Aminopyrrolidonderivat, Piperidinderivat, Aminoazaheterocyclus oder Isoindolderivat ist.

7. Verwendung nach einem der vorhergehenden Ansprüche, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

8. Verwendung nach einem der vorhergehenden Ansprüche, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

9. Verwendung nach einem der vorhergehenden Ansprüche, dadurch gekennzeichnet, daß das kosmetisch akzeptable Medium eine wäßrige oder wäßrig-alkoholische Lösung, eine Wasser-in-Öl-Emulsion, Öl-in-Wasser-Emulsion, Mikroemulsion, ein wäßriges Gel, ein wasserfreies Gel, ein Serum oder eine Vesikeldispersion ist.

10. Verwendung nach einem der vorhergehenden Ansprüche, dadurch gekennzeichnet, daß die Zusammensetzung mindestens ein Mittel enthält, das unter den antibakteriellen Mitteln, Mitteln gegen Parasiten, Mitteln gegen Pilze, Antiphlogistika, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren, ausgewählt ist.

11. Verwendung nach dem vorhergehenden Anspruch, dadurch gekennzeichnet, daß das Mittel das Lidocain-Hydrochlorid, ein Mittel gegen Parasiten oder ein nichtsteroides entzündungshemmendes Mittel ist.

12. Verwendung eines Antagonisten der Substanz P in einer kosmetischen Zusammensetzung zur Vorbeugung und/oder Bekämpfung von Hautreizungen und/oder Flechten und/oder Erythemen und/oder unangenehmen Empfindungen und/oder Hitzeempfindungen und/oder Juckreiz der Haut.

13. Verwendung nach Anspruch 12, dadurch gekennzeichnet, daß der Antagonist der Substanz P eine Substanz ist, die die Extravasation von Plasma durch die Gefäßwand vermindert, welche durch Capsaicin oder eine antidrome Nervenstimulierung hervorgerufen wird.

14. Verwendung nach Anspruch 12 oder 13, dadurch gekennzeichnet, daß der Antagonist der Substanz P eine Substanz ist, die eine Hemmung der durch die Verabreichung einer Substanz P hervorgerufenen Kontraktion der glatten Muskulatur hervorruft.

15. Verwendung nach einem der Ansprüche 12 bis 14, dadurch gekennzeichnet, daß der Antagonist der Substanz P unter den Peptiden und den Verbindungen, welche mindestens einen Stickstoffheterocyclus aufweisen, ausgewählt ist.

16. Verwendung nach einem der Ansprüche 12 bis 15, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

17. Verwendung nach einem der Ansprüche 12 bis 16, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

18. Verfahren zur kosmetischen Behandlung, dadurch gekennzeichnet, daß auf die Haut, das Haar und/oder die Schleimhäute der Personen mit empfindlicher Haut eine Zusammensetzung aufgetragen wird, welche den Antagonisten der Substanz P in einem kosmetisch akzeptablen Medium enthält.

19. Verfahren nach Anspruch 18, dadurch gekennzeichnet, daß der Antagonist der Substanz P eine Substanz ist, die die Extravasation von Plasma durch die Gefäßwand vermindert, die durch Capsaicin oder eine antidrome Nervenstimulierung hervorgerufen wird.

20. Verfahren nach Anspruch 18 oder 19, dadurch gekennzeichnet, daß der Antagonist der Substanz P eine Substanz ist, die eine Hemmung der durch die Verabreichung der Substanz P hervorgerufenen Kontraktion der glatten Muskulatur hervorruft.

21. Verfahren nach einem der Ansprüche 18 bis 20, dadurch gekennzeichnet, daß der Antagonist der Substanz P unter den Peptiden und den Verbindungen, welche mindestens einen Stickstoffheterocyclus aufweisen, ausgewählt ist.

22. Verfahren nach einem der Ansprüche 18 bis 21, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

23. Verfahren nach einem der Ansprüche 18 bis 22, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

24. Kosmetische Zusammensetzung, die ein kosmetisch akzeptables Medium und mindestens einen Wirkstoff mit reizender Nebenwirkung enthält, dadurch gekennzeichnet, daß sie einen Antagonisten der Substanz P enthält.

25. Kosmetische Zusammensetzung nach Anspruch 24, dadurch gekennzeichnet, daß der Antagonist der Substanz P eine Substanz ist, die die Extravasation von Plasma durch die Gefäßwand vermindert, die durch Capsaicin oder eine antidrome Nervenstimulierung hervorgerufen wird.

26. Kosmetische Zusammensetzung nach Anspruch 24 oder 25, dadurch gekennzeichnet, daß der Antagonist der Substanz P eine Substanz ist, die eine Hemmung der durch die Verabreichung der Substanz P hervorgerufenen Kontraktion der glatten Muskulatur hervorruft.

27. Kosmetische Zusammensetzung nach einem der Ansprüche 24 bis 26, dadurch gekennzeichnet, daß der Antagonist der Substanz P unter den Peptiden und den Verbindungen, die mindestens einen Stickstoffheterocyclus aufweisen, ausgewählt ist.

28. Kosmetische Zusammensetzung nach einem der Ansprüche 24 bis 27, dadurch gekennzeichnet, daß der Antagonist der Substanz P in einem Mengenanteil von 0,000001 bis 5 Gew.-%, bezogen auf das Gesamtgewicht der Zusammensetzung, verwendet wird.

29. Kosmetische Zusammensetzung nach einem der Ansprüche 24 bis 28, dadurch gekennzeichnet, daß der Antagonist der Substanz P in einem Mengenanteil von 0,0001 bis 0,1 Gew.-%, bezogen auf das Gesamtgewicht der Zusammensetzung, verwendet wird.

30. Kosmetische Zusammensetzung nach einem der Ansprüche 24 bis 29, dadurch gekennzeichnet, daß der Wirkstoff mit reizender Nebenwirkung ausgewählt ist unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, grenzflächenaktiven Stoffen, Parfums und Konservierungsmitteln.

31. Kosmetische Zusammensetzung nach einem der Ansprüche 24 bis 30, dadurch gekennzeichnet, daß sie ferner mindestens ein Mittel enthält, das ausgewählt ist unter den antibakteriellen Mitteln, Mitteln gegen Parasiten, Mitteln gegen Pilze, Antiphlogistika, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren.

32. Verwendung von Capsaicin zur Herstellung einer Zusammensetzung, die zur Bestimmung von empfindlicher Haut dient.

33. Zusammensetzung zur Reinigung und/oder zum Schutz und/oder zur Behandlung und/oder zur Pflege des Gesichtes und/oder der Hände und/oder der Füße und/oder der Körperfalten und/oder des Körpers und/oder zum Schminken und/oder zum Anschminken und/oder zur Anwendung nach dem Sonnenbad und/oder zur Selbstbräunung und/oder für das Bad und/oder zur Desodorisierung und/oder Epilation und/oder zur Anwendung nach der Rasur und/oder gegen Insektenstiche und/oder als schmerzstillende Zusammensetzung und/oder als Parfumzusammensetzung und/oder als Haarwaschmittel und/oder zur Haarpflege und/oder zur Haarbehandlung und/oder zur Anwendung im Mund-Zahn-Bereich, dadurch gekennzeichnet, daß sie einer Zusammensetzung nach einem der Ansprüche 24 bis 31 entspricht.

34. Kosmetische Zusammensetzung nach einem der Ansprüche 24 bis 31 oder 33, dadurch gekennzeichnet, daß das kosmetisch akzeptable Medium eine wäßrige oder wäßrig-alkoholische Lösung, eine Wasser-in-Öl-Emulsion, eine Ölin-Wasser-Emulsion, eine Mikroemulsion, ein wäßriges Gel, ein wasserfreies Gel, ein Serum oder eine Vesikeldispersion ist.

35. Kosmetische Zusammensetzung nach einem der Ansprüche 24 bis 31, 33 oder 34, dadurch gekennzeichnet, daß sie Zusatzstoffe enthält, wie hydrophile oder lipophile Gelbildner, hydrophile oder lipophile Wirkstoffe, Antioxidantien, Lösungsmittel, Füllstoffe, Filter und Färbemittel.
